# EUROPEAN PATENT SPECIFICATION

(11) **EP 1 779 788 B1**
(45) Date of publication and mention of the grant of the patent: **07.05.2008**
(21) Application number: 05023728.8
(22) Date of filing: 28.10.2005
(51) Int. Cl.: A61B 17/00, A61B 17/88

(54) **Suction fixing device**
Unterdruck-Haltevorrichtung
Dispositif de maintien par succion

(43) Date of publication of application: 02.05.2007
(73) Proprietor: BrainLAB AG, 85551 Heimstetten (DE)
(72) Inventor: Götte, Hubert, 80333 Munich (DE); Krugmann, Jens, 81667 Munich (DE)
(74) Representative: Schwabe - Sandmair - Marx

(56) References cited:
- EP-A- 0 797 958
- EP-A- 1 371 340
- US-A1- 2002 049 369
- US-A1- 2004 176 659
- US-A1- 2005 033 322

## Description

The present invention relates to a suction fixing device to be fixed on an a bone structure. In particular, the invention is directed to fixing a reference or marker element to a bone structure.

In recent years, surgical operation techniques have developed towards minimally invasive operating methods. These methods are often based on image-aided or computer-aided surgery. Such surgical methods need marker or reference elements or arrays to enable the accuracy necessary for minimally invasive operating techniques. During a surgical operation, it is necessary for the reference or marker elements to not be able to be misplaced, since accuracy is closely related to maintaining orientation in the surgical operating space. Therefore, the reference or marker elements or arrays have to be fixed rigidly and reliably. On the other hand, it is necessary for the reference or marker elements to be able to be fixed to the organic base, in particular a bone structure, and to also be able to be detached with a minimally invasive influence on the organic base. In the prior art, screws or nails are commonly used and the bone has to be penetrated by these surgical tools. Accordingly, serious injury is caused to the bone structure and it is possible for the bone structure to be additionally weakened, in particular if the bone is spelled as is for instance possible in the case of elderly patients, partially destroyed bones and the like.

US 2004/176659A discloses an organ manipulator with at least one suction member or adhesive disc to be connected with the organ to be manipulated. The manipulator is provided with at least one compliant joint to allow a free movement. This organ manipulator is provided for soft tissue like a beating heart.

US 2002/049369 A discloses a heart retractor for use in heart surgery and is only intended for soft tissue.

EP 1 371 340 A proposes to fix some marker elements also to a bone structure and proposes to use a suction device which is intended for soft tissue applications.

Accordingly, it is the object of the present invention to provide another minimally invasive tool for the corresponding surgical operating techniques.

Furthermore, it is another object of the present invention to provide a new surgical tool which does not need to penetrate into a bone structure, in order to be securely fixed to the organic base.

It is another object of the present invention to provide a new surgical tool which can be attached and detached very quickly. Another object is to provide a surgical tool which allows a small area of contact, in particular on a bone structure, to be used for securely fixing to said bone structure.

At least one of the above objects is at least partially solved by a suction fixing device according to claim 1.

At least some of the advantages of the present invention are based on a suction fixing device including at least one low-pressure casing, at least one attachment device for applying a low pressure to a surface of a bone structure, and at least one valve means for connecting an evacuation device and/or at least one integrated evacuation device, said evacuation device generating a low pressure in said low-pressure casing.

Using the suction fixing device according to the invention, it is possible to attach the device to a small area in the surgical field, i.e. the bone structure, very quickly and also to detach the device according to the invention very quickly, by releasing a low pressure in the low-pressure casing. The attachment device of the suction fixing device can be attached to a bone structure such that a low pressure can be generated and maintained in the low-pressure casing, such that atmospheric pressure presses the suction fixing device against the organic base. The device can thus be securely fixed using a simple surgical tool. It is not then possible to misplace a marker or reference element or array fixed to the suction fixing device according to the invention.

The valve means can be used to connect an evacuation device to the suction fixing device according to the invention, in order to generate the low pressure in the low-pressure casing.

A syringe can for instance be used to lower the pressure in the low-pressure casing. Another way of generating a low pressure is to use an integrated evacuation device, which means that the evacuation device, for instance a suction piston, syringe or the like, can be part of the suction fixing device according to the invention. It can for instance be directly connected to the low-pressure casing. In this case, it is preferable if the piston rod can be locked or blocked, to avoid atmospheric pressure pressing the piston rod into the piston volume, thereby releasing the low pressure in the piston volume. It is also possible to connect an electrical valve pump to the valve means, wherein said valve pump can be disconnected once the low pressure has been generated in the low-pressure casing. To detach the suction fixing device according to the invention, it is merely necessary to open the valve means or to release the syringe, in order to equalise the low pressure in the low-pressure casing.

It is also possible that the edge of the suction fixing device be bend to adjust the suction fixing device or suction device also to more complicated surfaces of a bone.

According to the present invention, a holding structure is formed close to said attachment device. This holding structure can be adjusted so as to be effective in the plane of the attachment device used to apply the low pressure to the surface of the bone structure. Accordingly, if the attachment device, for instance a suction cup, is urged against the bone structure the holding structure can be urged against the surface of the bone structure and can secure the suction fixing device against shifting forces and/or torques applied to it. Accordingly, the holding structure can have a structure such as a spiked or ridged structure, wherein said spikes or ridges or the like are urged against the surface of the bone structure when generating the low pressure in the low-pressure casing, which in turn urges the suction fixing device according to the invention against the surface of the bone structure.

It is also possible to compensate a tilt angle between the suction cup on the holding structure for instance by means of elongating or shortening the holding structure on one or several sides thereof. For example, in case the holding structure includes some pin-shaped legs, in particular three pin-shaped legs, it is possible to elongate or shorten at least one of the legs to adjust the plane stretched by the ends of the legs to the plane of the suction cup.

According to another advantageous embodiment, a joint element is provided for adjustably fixing surgical equipment to the suction fixing device of the invention. A reference or marker element or array can for instance be fixed to the joint element. Such a reference or marker element or array can be used in image guided surgical methods or computer aided surgical methods, e.g. in connection with Vector Vision/Trauma 2.5, both products of the applicant.

According to another advantageous embodiment, a flexible connection can be interposed between the attachment device, i.e. a suction cup, and the evacuation device and/or the valve means. If the suction fixing device according to the invention is slightly inclined while being fixed to the surface of the bone structure, it is possible for internal stress, internal torques and the like to be exerted on the suction fixing device according to the invention. This additional strain could loosen or contribute to loosening the suction fixing device according to the invention. However, the flexible connection can be used to equalise such inclinations, torques or the like.

According to another advantageous embodiment, a frame is provided which supports at least some of the parts of the suction device. The frame can support the low-pressure casing, the attachment device, the valve means or syringe, the evacuation device and so on, such that the mechanical stability and thus the ability to securely fix the suction fixing device according to the invention to a bone structure can be additionally improved. Furthermore, the holding device can be formed at the end of the frame, such that the holding device is directed towards the surface of the bone structure.

Furthermore, a joint element can be provided to absorb further strains, inclinations or torques and also shifting forces such that a part of the suction fixing device according to the invention can be pivoted or rotated with respect to another part of it. The function of the joint element can be assisted by the holding structure and the other way round.

To enable the necessary degree of freedom, the frame can be provided with at least one recess for leading the connection or flexible connection through which is preferably part of the low-pressure volume associated with the low-pressure casing. The recess can have larger dimensions than the connection or flexible connection, to allow the intended additional degree of freedom, in particular for movements by a joint element such as a ball joint.

In accordance with the invention, it is possible to fix a marker array to the bone structure without using penetrating surgical tools. Thus, the bone structure is not injured and the bone surface does not have to be penetrated.

According to the invention, it is possible for example to use a femoral marker array to pivot the hip centre point. It is also possible, for instance, to use the device according to the invention to attach a reference array as a reference when acquiring surface points in a gap of a joint in a human or animal body. The suction fixing device of the invention can be used to serve these purposes by attaching it to the cartilage surface of the joint end of the femur, after opening a knee joint. Accordingly, the suction fixing device according to the invention can be used in connection with most hip and knee applications, at least where computer-aided surgical techniques are used.

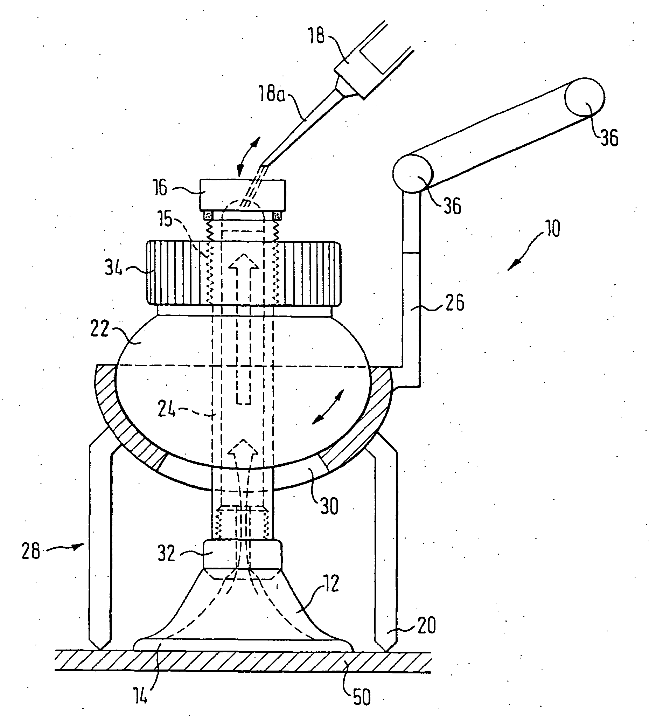

A preferred embodiment according to the present invention will now be described with reference to the single figure attached, which shows a single principle view, partly in a sectional view and partly in a perspective view.

Specifically, one embodiment of the invention includes a suction cup and a marker array mounted to the suction cup. The suction cup can be provided with a vacuum source such as an electrical vacuum pump or a mechanical suction device such as a syringe. The vacuum source can also be incorporated into the device according to the invention. The piston rod can be formed as a screw which, if rotated, pulls the piston out of the evacuation device and sucks air or gas out of the suction cup. While in most cases, it seems to be sufficient to simply use the suction cup as the low-pressure casing in connection with the attachment device, in some cases in which it is complicated to fix the suction fixing device by means of the force exerted by atmospheric pressure only, a holding device with pins, spikes, friction ridges or the like can also be used. The suction cup can be partially or completely made of silicone, rubber or the like. In addition, it is possible for the suction cup to be made of metal or glass and for only a suction lip provided to be made of silicone, rubber or the like. It is also possible to use more the one suction cup, e.g. two or three suction cups, wherein the other suction cup or cups are contacting the bone or joint structure at other locations, for instance at a rotationally shifted and/or linearly shifted position with respect to the first suction cup.

In the figure, a preferred embodiment of the suction fixing device according to the present invention is designated as a whole by the reference number 10. The device 10 includes a low-pressure casing 12 which can for example be a suction cup. The low-pressure casing includes an attachment device 14 which is designed to be brought into close contact with a surface of a bone structure 50. The attachment device 14 can for instance include a flexible lip made of rubber, silicone or the like, to be attached to the bone surface such that a low-pressure atmosphere in the low-pressure casing 12 can be maintained and is not released through a gap between a surface of the bone 50 and the attachment device 14. The edge of the attachment device with the corresponding lip can also have a bent shape. The low-pressure casing 12 is connected to a valve means or adapter means 16. This valve or adapter means can be used to connect an evacuation device 18, in particular a syringe 18 or an electrical vacuum pump (not shown), to the low-pressure casing 12. If a syringe 18 is used, the syringe can have a suction extension 18a to be coupled to the valve means or adapter means 16. If the syringe 18 remains connected to the adapter means 16, the piston rod (not shown) of the syringe 18 should be held in a fixed position, i.e. blocked in a low-pressure position such that the low-pressure atmosphere in the low-pressure casing 12 can be maintained over a long period of time.

The low-pressure casing 12 is connected by a connecting device 32 to a connection or flexible connection 24 which is connected in a seal to the valve or adapter means 16. A joint element 22, in the present case a ball joint, is provided in order to allow inclinations between the low-pressure casing 12, i.e. the attachment device 14, and a coupling element 26 which can support one or more reference or marker elements 36 which together can form a marker array. A frame 28 is provided to support all the different elements and parts of the suction fixing device 10 of the present invention and the joint element 22 also allows a tilt between the frame 28 and the low pressure casing 12 and the suction cup 12. A holding device 20 is also provided at the lower end of the frame 28. The holding device 20 consists of a number of pins or spikes or a friction frame, such that the lower end or ends of the holding device 20 are in the same plane as the attachment device or attachment lip 14, such that when atmospheric pressure presses the device 10 against the surface of a bone 50, the attachment device 14 is pressed with it, thus urging the tips of the pins of the holding device 20 against the surface of the bone. The pins of the holding device can also be elongated or shortened. This additionally helps the device 10 to resist shifting or rotational forces, such that the device 10 according to the invention can be made even more secure.

The frame 28 is also provided with one or more recesses. One of the recesses is a recess 30 which is provided in order to provide a lead-through for the connection line 24 between the low-pressure casing or suction cup 12 and the valve or adapter means 16. The recess 30 has larger dimensions and in particular a larger diameter than the diameter of the connection or flexible connection 24. This enables the joint element or joint 22 to rotate without being limited by the edges of the recess 30. The connection line 24 which can at least partial be flexible can be threaded at its upper end. This threaded upper end 15 can also be used for biasing the device 10 with respect to the bone or joint structure to which the device 10 just had been fixed by means of a low pressure in the suction cup 14. The fastening wheel 14 can be rotated fro screwing the flexible connection towards the bone 50 or away from the bone 50.

When using the device 10 of the present invention, a soft tissue portal has to be provided first in order to have access to the surface of a patient's bone. The surgical field, for instance a hip or inlay, is then prepared for a computer-aided or image-guided operating technique, wherein the device 10 is attached to the surface of the bone 50. A syringe 18 is then coupled to the adapter or valve means 16 and the piston or syringe 18 is actuated in order to withdraw air or gas from the low-pressure casing 12 so as to generate a low-pressure atmosphere in the low-pressure casing 12. Once a low-pressure atmosphere has been generated, atmospheric pressure presses the whole device 10 against the surface of bone 50, thereby urging the holding device 20, e.g. pins, spikes or ridges, against the surface of the bone 50. The reference or marker array 36 can then be adjusted to allow the computer-aided or image-guided operating technique. The valve or adapter means 16 can be fixed to the frame 28 or the joint element 22 by means of a fastening device or fastening wheel 34, such that the whole valve or adapter means 16, the flexible connection 24 and the low-pressure casing 12 with the attachment device 14 can be exchanged quickly.

### List of reference numerals

- 10: suction fixing device
- 12: low-pressure casing, suction cup
- 14: attachment device
- 15: threaded upper end
- 16: valve means, adapter means
- 18: evacuation device, syringe
- 18a: suction extension
- 20: holding device
- 22: joint element, e.g. ball joint
- 24: flexible connection
- 26: coupling element
- 28: frame
- 30: recess
- 32: connecting device
- 34: fastening device, fastening wheel
- 36: reference or marker elements
- 50: organic base; bone; bone surface

## Claims

1. A suction fixing device (10) to be fixed to a bone structure (50), comprising:
at least one low-pressure casing (12); and
at least one attachment device (14) for applying a low pressure to the surface of said bone (50); wherein said suction fixing device (10)
further comprises a holding or friction device (20) like a number of pins, spikes or a friction frame, which is positioned in such a way that the said holding or friction device (20) is urgeable against the surface of the bone (50) when vacuum is applied to the low-pressure casing (12).

2. The device according to claim 1, wherein the device further comprises:
- at least one valve means (16) for connecting an evacuation device (18) and/or at least one integrated evacuation device (18), said evacuation device generating a low pressure, in said low-pressure casing (12).

3. The device according to any one of claims 1 or 2, wherein the holding or friction structure (20) is formed close to said attachment device (14).

4. The device according to any one of claims 1 to 3, wherein a joint element (22) is provided for adjustably fixing surgical equipment (36) to the device (10), in particular for fixing a reference element or marker, a reference array (36) or the like, for computer-aided or image-guided surgery.

5. The device according to any one of claims 1 to 4, wherein a flexible connection (24) is interposed between the attachment device (14) and the evacuation device (18) and/or the valve or adapter means (16).

6. The device according to any one of claims 1 to 5, wherein the evacuation device is a syringe (18, 18a).

7. The device according to any one of claims 1 to 6, wherein a frame (28) is formed, supporting at least some of the parts of the device (10).

8. The device according to claim 7, wherein the joint element (22) and/or the attachment device (14) are connected to said frame (28).

9. The device according to claim 8, wherein the frame (28) is provided with a recess (30) for leading a connection or said flexible connection (24) through, preferably as part of a low-pressure volume associated with said low-pressure casing (12), said recess (30) having a larger dimension or larger dimensions than said connection or flexible connection (24) in order to allow a degree of freedom for the connection or flexible connection (24), in particular for movements by the joint element (22), in particular a ball joint (22).

## Patentansprüche

1. Eine Saug-Befestigungs-Vorrichtung (10), die an einer Knochenstruktur (50) befestigt werden soll, die folgendes aufweist:
wenigstens ein Niederdruck-Gehäuse (12); und
wenigstens eine Befestigungseinheit (14), um einen Niederdruck auf die Oberfläche des Knochens (50) auszuüben;
wobei die Saug-Befestigungs-Vorrichtung (10) des weiteren eine Halte- oder Reibungseinheit (20) wie eine Anzahl von Pins, Spikes oder einen Reibungsrahmen, aufweist, welche in einer solchen Weise positioniert ist, dass die Halte- oder Reibungseinheit (20) gegen die Oberfläche des Knochens (50) gedrängt werden kann, wenn ein Vakuum an der Niederdruckeinheit (12) angelegt wird.

2. Die Vorrichtung gemäß Anspruch 1, wobei die Vorrichtung des weiteren folgendes aufweist:
- wenigstens ein Ventilmittel (16) zum Verbinden eines Evakuierungsmittels (18) und / oder wenigstens ein integriertes Evakuierungsmittel (18), wobei das Evakuierungsmittel in dem Niederdruck-Gehäuse (12) einen Niederdruck erzeugt.

3. Die Vorrichtung gemäß irgend einem der Ansprüche 1 oder 2, wobei die Halte- oder Reibungsstruktur (20) nahe bei der Befestigungseinheit (14) ausgebildet ist.

4. Die Vorrichtung gemäß irgend einem der Ansprüche 1 bis 3, wobei ein Verbindungselement bzw. Gelenk (22) vorgesehen ist, um einstellbar bzw. anpassbar chirurgische Ausrüstung (36) an der Vorrichtung (10) zu befestigen, insbesondere um ein Referenzelement oder einen Marker, oder eine Referenzanordnung (36) oder dergleichen, für Computer-unterstützte oder Bild-geführte Operationen zu befestigen.

5. Die Vorrichtung gemäß irgend einem der Ansprüche 1 bis 4, wobei eine flexible Verbindung (24) zwischen dem Befestigungsmittel (14) und dem Evakuierungsmittel (18) und / oder dem Ventil oder Anpassungsmittel (16) eingefügt ist.

6. Die Vorrichtung gemäß irgend einem der Ansprüche 1 bis 5, wobei das Evakuierungsmittel eine Spritze (18, 18a) ist.

7. Die Vorrichtung gemäß irgend einem der Ansprüche 1 bis 7, wobei ein Rahmen (28) gebildet ist, der wenigstens einige der Teile der Vorrichtung (10) lagert.

8. Die Vorrichtung gemäß Anspruch 7, wobei das Verbindungselement bzw. Gelenk (22) und / oder das Befestigungsmittel (14) mit dem Rahmen (28) verbunden sind.

9. Die Vorrichtung gemäß Anspruch 8, wobei der Rahmen (28) mit einer Einbuchtung (30) versehen ist, um eine Verbindung oder die flexible Verbindung (24) hindurch zu führen, bevorzugt als Teil eines Niederdruck-Volumens, das mit dem Niederdruck-Gehäuse (12) verbunden ist, wobei die Einbuchtung (30) eine größere Abmessung bzw. Dimension oder größere Abmessungen bzw. Dimensionen hat als die Verbindung oder flexible Verbindung (24), um einen Freiheitsgrad bzw. Spielraum für die Verbindung oder flexible Verbindung (24) zu erlauben, insbesondere für Bewegungen durch das Verbindungselement bzw. Gelenk (22), insbesondere durch ein Kugelgelenk (22).

## Revendications

1. Dispositif de fixation par aspiration (10) destiné à être fixé à une structure d'os (50), comprenant :
au moins une enceinte à basse pression (12) ; et
au moins un dispositif de fixation (14) pour appliquer une basse pression à la surface dudit os (50) ;
dans lequel ledit dispositif de fixation par aspiration (10) comprend de plus un dispositif de maintien ou de frottement (20), tel qu'un certain nombre de broches, de pointes ou un bâti de frottement, qui est positionné de telle sorte que ledit dispositif de maintien ou de frottement (20) puisse être poussé contre la surface de l'os (50) lorsqu'un vide est appliqué à l'enceinte à basse pression (12).

2. Dispositif selon la revendication 1, dans lequel le dispositif comprend de plus :
- au moins un moyen formant vanne (16) pour relier un dispositif d'évacuation (18) et/ou au moins un dispositif d'évacuation intégré (18), ledit dispositif d'évacuation générant une basse pression, dans ladite enceinte à basse pression (12).

3. Dispositif selon l'une quelconque des revendications 1 ou 2, dans lequel la structure de maintien ou de frottement (20) est formée à proximité dudit dispositif de fixation (14).

4. Dispositif selon l'une quelconque des revendications 1 ou 3, dans lequel un élément de raccord (22) est disposé pour fixer de façon réglable un équipement chirurgical (36) au dispositif (10), en particulier pour fixer un élément de référence ou de repérage, un système de référence (36), ou analogue, pour une chirurgie aidée par ordinateur ou guidée par image.

5. Dispositif selon l'une quelconque des revendications 1 à 4, dans lequel un raccordement souple (24) est interposé entre le dispositif de fixation (14) et le dispositif d'évacuation (18) et/ou les moyens formant vanne ou adaptateur (16).

6. Dispositif selon l'une quelconque des revendications 1 à 5, dans lequel le dispositif d'évacuation est une seringue (18, 18a).

7. Dispositif selon l'une quelconque des revendications 1 à 6, dans lequel un bâti (28) est formé, supportant au moins certaines des parties du dispositif (10).

8. Dispositif selon la revendication 7, dans lequel l'élément de raccord (22) et/ou le dispositif de fixation (14) sont reliés audit bâti (28).

9. Dispositif selon la revendication 8, dans lequel le bâti (28) est muni d'une cavité (30) pour mener un raccordement ou ledit raccordement souple (24) à travers, de préférence sous la forme d'une partie d'un volume à basse pression associé à ladite enceinte à basse pression (12), ladite cavité (30) ayant une dimension supérieure ou des dimensions supérieures à celles dudit raccordement ou dudit raccordement souple (24) afin d'autoriser un degré de liberté pour le raccordement ou le raccordement souple (24), en particulier pour des mouvements de l'élément de raccord (22), en particulier un joint à rotule (22).
